# EUROPEAN PATENT SPECIFICATION

(11) **EP 4 281 046 B1**
(45) Date of publication and mention of the grant of the patent: **10.09.2025**
(21) Application number: 22702657.2
(22) Date of filing: 25.01.2022
(51) Int. Cl.: A61K 9/20, A61K 9/24, A61K 9/19, A61K 31/485, A61K 31/593, A61P 35/00

(54) **NALTREXONE COMPOSITIONS**
NALTREXONZUSAMMENSETZUNGEN
COMPOSITIONS À BASE DE NALTREXONE

(30) Priority: 25.01.2021 EP 21153332
(43) Date of publication of application: 29.11.2023
(73) Proprietor: LDN Pharma Limited, London EC2V 7BG (GB)
(72) Inventor: HOOD, Francis, London EC2R 6AY (GB)
(74) Representative: Gill Jennings & Every LLP
(86) International application number: PCT/EP2022/051619
(87) International publication number: WO 2022/157385

(56) References cited:
- WO-A1-2008/127669
- WO-A1-2019/186207
- WO-A1-2020/178447
- WO-A1-2020/188255
- WO-A2-2007/036802
- WO-A2-2017/141104
- CN-A- 109 316 454
- US-A1- 2020 138 721
- US-A1- 2020 146 993
- YINGYU MA ET AL: "Vitamin D in combination cancer treatment", JOURNAL OF CANCER, vol. 1, 15 July 2010 (2010-07-15), pages 101 - 107, XP055061348, ISSN: 1837-9664, DOI: 10.7150/jca.1.101

## Description

### Field of the invention

The present invention is directed to a single unit oral dose pharmaceutical composition comprising naltrexone and calcitriol for use in the treatment of various disorders. such as cancer.

### Background of the invention

The success of many cancer therapies is predicated by co-administration alongside adjuvant-type molecules. Without any independent therapeutic utility, adjuvants are responsible for priming the immune system of a subject such that the active compound targeting the cancer can achieve maximum therapeutic effect.

As adjuvants typically modulate the immune response of a patient, they are used most commonly in conjunction with cancer therapies or biologics such as humanized therapeutic antibodies. They act either to enhance the immune system of a patient to increase the production of antibodies in response to challenge with a cancer therapies, or by supressing or lowering the immunogenicity of the patient towards a foreign therapeutic antibody. Thus, adjuvants play an important role in driving immune cancer therapies towards a successful therapeutic outcome.

Often, immunotherapies will be combined with more traditional cancer therapies such as radiotherapy or chemotherapy. For certain types of cancers where there exists no efficacious immunotherapy, only traditional therapies can be administered. Traditional cancer treatments can also be administered in combination, where the therapeutic effect can be greater upon co-administration than the sum of the effects upon independent administration.

Despite the greater efficacy, the combination of traditional cancer therapies can exacerbate side-effects experienced by the patient, often resulting in early termination of the treatment regimen. Thus the beneficial synergistic effect of coadministering multiple anti-cancer agents can go unrealised due to the harsh nature of the therapy.

The development of new treatments with greater efficacy and reduced side effects would circumvent the need to co-administer certain cancer therapies, thus avoiding the harsh side-effects that often lead to premature treatment termination. Alternatively, the development of adjuvant-like molecules that boost the therapeutic efficacy of chemotherapeutic agents would achieve a similar outcome.

Thus, there is a need to develop agents that boost the therapeutic utility of chemotherapeutic agents in order to minimize the detrimental side effects of what would otherwise be aggressive cancer treatment regimens.

Furthermore, there is a need to administer such agents to patients in a manner that maximises therapeutic effect and minimises any adverse effects or issues. Oral dosage forms such as tablets or capsules to be swallowed by the patient are commonly used. However, one major limitation is lack of bioavailability of the active ingredient through this means of administration (i.e. by swallowing).

Thus, there is a need to develop agents that boost the therapeutic utility of chemotherapeutic agents, in a formulation that maximises bioavailability of the active ingredient.

WO 2019/186207 discloses the administration of naltrexone and vitamin D for use in the treatment of autoimmune diseases.

### Summary of the invention

According to a first aspect of the invention, there is provided a single unit oral dose pharmaceutical composition comprising a first active ingredient in a first formulation and a second active ingredient in a second formulation;
wherein the first active ingredient is for absorption in the gastrointestinal tract from the oesophagus onwards;
wherein the second active ingredient is formulated for absorption in the oral cavity;
wherein the first active ingredient is naltrexone in an amount of 0.01 to 10 mg and the second active ingredient is calcitriol in an amount of 80 to 200 ug;
wherein the first formulation comprising naltrexone is in the form of a tablet and wherein the second formulation comprising calcitriol is a lyophilised liquid solution, suspension or emulsion which is attached to the tablet;
wherein the first formulation comprising naltrexone in the form of a tablet is formulated for delayed release; and
wherein the first formulation comprising naltrexone is formulated for release and/or absorption after the second formulation comprising calcitriol is released and/or absorbed.

According to a second aspect of the invention, there is provided a single unit oral dose pharmaceutical composition comprising a first active ingredient in a first formulation and a second active ingredient in a second formulation for use as a medicament;
wherein the first active ingredient is for absorption in the gastrointestinal tract from the oesophagus onwards;
wherein the second active ingredient is formulated for absorption in the oral cavity;
wherein the first active ingredient is naltrexone in an amount of 0.01 to 10 mg and the second active ingredient is calcitriol in an amount of 80 to 200 ug;
wherein the first formulation comprising naltrexone is in the form of a tablet and wherein the second formulation comprising calcitriol is a lyophilised liquid solution, suspension or emulsion which is attached to the tablet;
wherein the first formulation comprising naltrexone in the form of a tablet is formulated for delayed release; and
wherein the first formulation comprising naltrexone is formulated for release and/or absorption after the second formulation comprising calcitriol is released and/or absorbed.

According to a third aspect of the invention, there is provided a single unit oral dose pharmaceutical composition comprising a first active ingredient in a first formulation and a second active ingredient in a second formulation for use in the treatment of cancer within a subject;
wherein the first active ingredient is for absorption in the gastrointestinal tract from the oesophagus onwards;
wherein the second active ingredient is formulated for absorption in the oral cavity;
wherein the first active ingredient is naltrexone in an amount of 0.01 to 10 mg and the second active ingredient is calcitriol in an amount of 80 to 200 ug;
wherein the first formulation comprising naltrexone is in the form of a tablet and wherein the second formulation comprising calcitriol is a lyophilised liquid solution, suspension or emulsion which is attached to the tablet;
wherein the first formulation comprising naltrexone in the form of a tablet is formulated for delayed release; and
wherein the first formulation comprising naltrexone is formulated for release and/or absorption after the second formulation comprising calcitriol is released and/or absorbed.

### Description of the drawings

Only the single unit oral dose pharmaceutical compositions defined in the claims are according to the invention.
Figures 1A and 1B show the effect of naltrexone (LDN) and calcitriol (CCT), in combination with the chemotherapeutic agent oxaliplatin on HCT116 (colorectal cancer) cell numbers and cell viability.
Figures 2A and 2B show the effect of naltrexone (LDN) and calcitriol (CCT), in combination with the chemotherapeutic agent oxaliplatin on A549 (lung cancer) cell numbers and cell viability.
Figures 3A and 3B show the effect of naltrexone (LDN) and calcitriol (CCT), in combination with the chemotherapeutic agent gemcitabine on HCT116 (colorectal cancer) cell numbers and cell viability.
Figures 4A and 4B show the effect of naltrexone (LDN) and calcitriol (CCT), in combination with the chemotherapeutic agent gemcitabine on A549 (lung cancer) cell numbers and cell viability.

### Detailed description of the invention

The inventors discovered that a single unit oral dose pharmaceutical composition comprising a first active ingredient in a first formulation and a second active ingredient in a second formulation;
wherein the first active ingredient is for absorption in the gastrointestinal tract from the oesophagus onwards;
wherein the second active ingredient is formulated for absorption in the oral cavity;
wherein the first active ingredient is naltrexone in an amount of 0.01 to 10 mg and the second active ingredient is calcitriol in an amount of 80 to 200 ug;
wherein the first formulation comprising naltrexone is in the form of a tablet and wherein the second formulation comprising calcitriol is a lyophilised liquid solution, suspension or emulsion which is attached to the tablet;
wherein the first formulation comprising naltrexone in the form of a tablet is formulated for delayed release; and
wherein the first formulation comprising naltrexone is formulated for release and/or absorption after the second formulation comprising calcitriol is released and/or absorbed
is optimised for delivery of its actives for therapeutic utility, particularly when used as an agent that boosts the therapeutic utility of anti-cancer agents.

It has been found that the combination of naltrexone and calcitriol in a single unit oral dose can be administered to patients as part of a treatment for cancer, irrespective of renal or liver function. The benefit of this is that the patients can be prescribed the single unit dose without the requirement for pre-screening for renal or liver function/dysfunction. The clinician therefore has confidence that an adequate dose of the medicaments is being prescribed with minimal potential side-effects.

The inventors have realised the benefit of combining low doses of naltrexone and calcitriol, in forms that provide different absorption rates, such that the components act in a synergistic manner to aid the desired therapeutic effect.

The single unit oral dose of the present invention allows for different, optimised routes of delivery of the two active ingredients: CCT is absorbed in the oral cavity and naltrexone is absorbed beyond the oral cavity i.e. in the gastrointestinal tract from the oesophagus onwards after being swallowed by the patient. The inventors have found that this results in improved bioavailability of the active ingredients. The inventors have achieved this improved bioavailability in a single unit dose, which is simple and convenient for patients to take and therefore leads to improved patient compliance.

Calcitriol is a metabolite of vitamin D. The normal metabolism process of vitamin D is that it is first converted into calcifediol (CCD) in the liver and then may be converted to calcitriol (CCT) in the kidneys. CCD has a long half-life and persists in the blood. However, when CCT is needed, CCD is converted to CCT in the kidneys. This essentially means that the concentration of CCT is kept relatively constant due to tightly-controlled regulation by the body. Typically, the plasma concentration is maintained around 60pg/ml. Increasing the supplementation of vitamin D does not tend to significantly increase the concentration of CCT due to this regulation process.

Certain cancer patients may have impaired renal and liver function. Certain cancer cells have elevated CYP enzymes which means vitamin D metabolism is compromised. Specifically, CCT cannot be converted from its precursor CCD as it is unable to be converted from vitamin D. In addition, patients with liver or kidney cancer may also be unable to metabolise vitamin D in the normal way, and so CCT cannot be converted.

Therefore, the present inventors identified that it would be desirable to administer CCT specifically to patients with cancer, rather than administer vitamin D.

However, the present inventors noted that, when swallowed by the patient, for example in a tablet, CCT has variable bioavailability which means plasma concentrations can vary. This variability means that the desired dosage may not be achieved. One way to mitigate this problem is to increase the dose per tablet to give the top-end of the dose. However, by doing this, there would be a danger of causing renal damage from toxicity.

The single unit oral dose of the present invention allows for CCT to be absorbed in the oral cavity which has been found to have a more consistent delivery of CCT. Furthermore, the single unit oral dose of the present invention allows for naltrexone to be absorbed beyond the oral cavity i.e. in the gastrointestinal tract from the oesophagus onwards which results in optimum delivery of naltrexone.

Thus the present inventors have identified a composition that is optimised for delivery of its actives in the treatment of cancer, particularly in combination with the use of anti-cancer agents. The composition can be easily administered to patients as a single unit oral dose, whilst maximising the bioavailability of the active ingredients.

In a first aspect of the invention, there is provided a single unit oral dose pharmaceutical composition comprising a first active ingredient in a first formulation and a second active ingredient in a second formulation;
wherein the first active ingredient is for absorption in the gastrointestinal tract from the oesophagus onwards;
wherein the second active ingredient is formulated for absorption in the oral cavity;
wherein the first active ingredient is naltrexone in an amount of 0.01 to 10 mg and the second active ingredient is calcitriol in an amount of 80 to 200 ug;
wherein the first formulation comprising naltrexone is in the form of a tablet and wherein the second formulation comprising calcitriol is a lyophilised liquid solution, suspension or emulsion which is attached to the tablet;
wherein the first formulation comprising naltrexone in the form of a tablet is formulated for delayed release; and
wherein the first formulation comprising naltrexone is formulated for release and/or absorption after the second formulation comprising calcitriol is released and/or absorbed.

As used herein the term "pharmaceutical composition" means, for example, a mixture containing a specified amount of a therapeutic compound or compounds, e.g. a therapeutically effective amount, in a pharmaceutically acceptable carrier to be administered to a mammal, e.g., a human in order to treat a disease.

As used herein the term "pharmaceutically acceptable" refers to those compounds, materials, compositions and/or dosage forms, which are, within the scope of sound medical judgment, suitable for contact with the tissues of mammals, especially humans, without excessive toxicity, irritation, allergic response and other problem complications commensurate with a reasonable benefit/risk ratio.

As used herein the term "single unit oral dose" has its normal meaning in the art. It may also be called an oral dosage form. It may, for example, be a tablet, a coated tablet, a bi-layered tablet, or a multi-layered tablet.

The single unit oral dose pharmaceutical composition comprises naltrexone as the first active ingredient.

As used herein "naltrexone" refers to morphinan-6-one,17-(cyclopropylmethyl)-4,5-epoxy-3,14-dihydroxy-(5α). Its empirical formula is C₂₀H₂₃NO₄ and its molecular weight is 341.41 in the anhydrous form (<1% maximum water content). The chemical structure of naltrexone is:

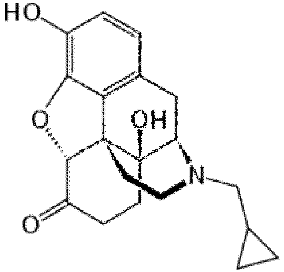

The single unit oral dose pharmaceutical composition comprises naltrexone in an amount of 0.01 to 10 mg. Preferably, the single unit oral dose comprises an amount of 0.1 mg to 6 mg, more preferably 1 mg to 5 mg, most preferably 3 to 4.5 mg of naltrexone. The dosage of naltrexone has been found to be particularly effective in acting in conjunction with anti-cancer agents.

The single unit oral dose pharmaceutical composition is formulated such that the first active ingredient, naltrexone, is for absorption in the gastrointestinal tract from the oesophagus onwards. By this it is meant that naltrexone is absorbed for delivery into the blood stream in the oesophagus, stomach, small intestine, or large intestine, (i.e. the gastrointestinal tract starting from the oesophagus and following its natural course). Naltrexone is not substantially absorbed in the patient's oral cavity.

Absorption of naltrexone into the gastrointestinal tract from the oesophagus onwards is achieved by the patient swallowing the single unit dose.

The single unit oral dose pharmaceutical composition comprises calcitriol as the second active ingredient.

As used herein, "calcitriol" refers to the active metabolite of vitamin D, specifically vitamin D3. Calcitriol is also called 1,25-dihydroxyvitamin-D3 or 1,25-dihydroxycholecalciferol. Its empirical formula is C₂₇H₄₄O₃. Its chemical structure is:

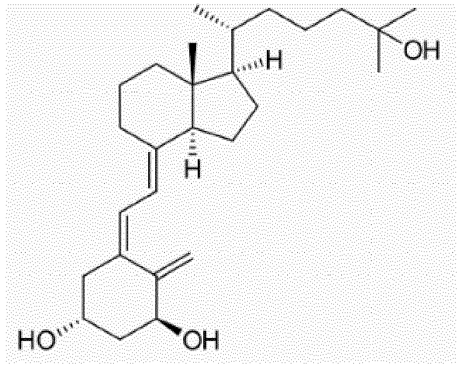

The single unit oral dose pharmaceutical composition comprises calcitriol in an amount of 80 to 200 ug. Preferably, the single unit oral dose comprises 90 to 190 ug, more preferably 100 to 180 ug, most preferably 110 to 170 ug. The dosage of naltrexone has been found to be particularly effective in acting in conjunction with anti-cancer agents.

The single unit oral dose comprising 80 to 200 ug calcitriol preferably achieves systemic (serum) concentration of calcitriol of 0.1 to 10 ng/ml, more preferably 2 to 8 ng/ml, most preferably 4 to 6 ng/ml.

The single unit oral dose pharmaceutical composition is formulated such that the second active ingredient, calcitriol, is for absorption in the oral cavity. The term oral cavity has its normal meaning in the art and is intended to cover the mouth, the lips, the hard palate, the soft palate, the retromolar trigone, the tongue, gingiva (gums), buccal mucosa (the inner lining of the lips and cheeks), and floor of the mouth under the tongue.

Absorption of calcitriol in the oral cavity may be achieved by the sublingual, sublabial, gingival or buccal route.

The single unit oral dose pharmaceutical composition according to the present invention comprises the naltrexone in a first formulation and the calcitriol in a second formulation. The first and second formulations are separate from each other, i.e. are not admixed. The first formulation is a tablet formulation, and the second formulation, as defined in the claims, is one that permits release and subsequent absorption of the calcitriol in the oral cavity.

The term formulation is intended to include the mixture of the active component(s) with any pharmaceutically acceptable excipients.

According to the invention, the first formulation comprising naltrexone is formulated as a tablet.

The tablet is administered orally and swallowed by the subject. By this it is meant that the subject swallows the tablet which is then metabolised through the traditional route, i.e. in the gastrointestinal tract, from the oesophagus onwards.

The tablet may be provided as a blend of both the naltrexone product and a combination of pharmaceutically acceptable excipients. As used herein, the term "excipient" refers to a pharmaceutically acceptable ingredient that is commonly used in pharmaceutical technology for the preparation of solid oral dosage formulations. Examples of categories of excipients include, but are not limited to, binders, disintegrants, lubricants, glidants, stabilizers, fillers, and diluents. The amount of each excipient used may vary within ranges conventional in the art.

Suitable excipients include magnesium carbonate, magnesium stearate, talc, lactose, lactose monohydrate, sugar, pectin, dextrin, starch, tragacanth, microcrystalline cellulose, methyl cellulose, sodium carboxymethyl cellulose, corn starch, colloidal anhydrous Silica, titanium dioxide, a low-melting wax, cocoa butter, and the like.

The first formulation may comprise components which allow for fast or sustained release of the first active ingredient, naltrexone, in the gastrointestinal tract from the oesophagus onwards. This can be achieved by including pharmaceutically acceptable rate controlling polymers. The rate controlling polymers may be hydrophilic or hydrophobic. Preferably the rate controlling polymers are selected from polyvinyl acetate, cellulose acetate, cellulose acetate butyrate, cellulose acetate propionate, ethyl cellulose, a fatty acid, a fatty acid ester, an alkyl alcohol, microcrystalline cellulose, a poly(meth)acrylate, a poly(ethylene oxide), polyuronic acid salts, cellulose ethers, xanthum gum, tragacanth gum, gum karaya, guar gum, acacia, gellan gum, locust bean gum, alkali metal salts of alginic acid or pectin acid, sodium alginate, potassium alginate, ammonium alginate, hydroxypropyl cellulose, hydroxyl ethyl cellulose, hydroxypropyl ethyl cellulose, carboxyvinyl polymer, polymerised gelatin and/or mixtures thereof.

The second active ingredient, calcitriol, is formulated for absorption in the oral cavity. In a preferred embodiment, the second formulation comprising calcitriol is formulated for absorption by the sublingual route. Absorption in the oral cavity can be achieved according to any known methods. For example, it can be formulated in an orally disintegrating form that disintegrates and dissolves in the mouth without water before swallowing. It may dissolve in this way within 60 seconds or less, preferably less than 10 seconds.

Preferably, the second formulation comprising calcitriol is an orally disintegrating tablet or coating, an orodisperse tablet or coating, a mouth-dissolving tablet or coating, a quick-dissolve tablet or coating, a fast-melt tablet or coating, a rapid-disintegrating tablet or coating; or a freeze-dried wafer.

An orally disintegrating tablet/coating is a solid dosage form that contains medicinal substances and disintegrates rapidly (within seconds) without water when placed on the tongue. The drug is released, dissolved, or dispersed in the saliva.

A quick-dissolving tablet/coating (also known as a fast-dissolving, fast-dissolving multiparticulate, rapid-dissolving, mouth-dissolving, fastmelting, or orodispersing tablets) is a solid dosage form that does not require water for swallowing. The tablet dissolves within 60 seconds when placed in the mouth. The active ingredients are absorbed through mucous membranes in the mouth.

A freeze-dried wafer is a quick-dissolving, thin matrix that contains a medicinal agent (in this case calcitriol) that does not need water for swallowing. The wafer disintegrates instantaneously in the oral cavity and releases calcitriol, which dissolves or disperses in the saliva.

This second formulation may be administered sublingually, sublabially or buccally to the subject by rapidly dissolving in the oral cavity when it comes in contact with saliva. Preferably, it is absorbed sublingually.

The second formulation comprising calcitriol is formulated according to the method of lyophilisation (freeze-drying), as a lyophilised dosage form selected from a lyophilised liquid solution, suspension or emulsion, and it is attached to the tablet of naltrexone. The second formulation can be achieved according to any known methods for producing dosage forms that dissolve in the oral cavity such as using the following technology: Zydis^{®} , QuickSolv^{®} , Lyoc^{®}, Flashdose^{®} , OraSolve^{®} , Ziplet technology, Frosta^{®} , DuraSolve^{®} , Wowtab^{®}, Durasolv^{®}, Flashtab^{®}, Oraquick^{®}, RapiTab^{®} and Nanomelt^{®} (by Elan).

The present inventors have discovered that the patient absorbing calcitriol in the oral cavity overcomes the problems of variable bioavailability that occurs when calcitriol is swallowed in a tablet. When swallowed, calcitriol has variable bioavailability which means the desired dosage may not be achieved. One way to mitigate this problem is to increase the dose per tablet to give the top-end of the dose. However, by doing this, there would be a danger of causing renal damage from toxicity. The present inventors have discovered that administering calcitriol in the oral cavity allows the desired dosage to be achieved without causing any adverse symptoms associated with renal damage, such as fatigue, loss of appetite or leg swelling. Absorption in the oral cavity also avoids drug metabolism via the stomach and intestines, which means a more efficient delivery of calcitriol.

The second formulation comprising calcitriol may also contain any conventional or pharmaceutically suitable additives suitable for providing absorption in the oral cavity. For example, it may include components that readily dissolve in saliva and contribute to the rapid disintegration in the mouth.

Preferred components may include matrix forming agents and sugars or sugar alcohols. These components are used to give sufficient strength to the unit dose to prevent breakage during removal and packaging, but once placed in the mouth, they allow immediate dissolution of the formulation.

Preferred matrix forming agents include gelatin, starches, modified starches, maltodextrin, cellulose gum, carrageenan gum, hyaluronic acid, pectins, carboxymethyl cellulose sodium, agar, gellan gum, guar gum, tragacanth gum, hydroxypropyl cellulose, hydroxyl propyl methylcellulose, methylcellulose, carbomer, poloxamer, polyacrylic acid, polyvinyl alcohol, alginates and polyglyconic acid or combinations thereof.

Preferred sugar alcohols include mannitol, sorbitol, glycerol, erythritol, maltitol, and lactitol or combinations thereof.

Preferred sugars include trehalose, dextrose, and lactose or combinations thereof.

In a preferred embodiment, the second formulation comprises gelatin and/or mannitol.

The second formulation comprising calcitriol is formed by cryogenically freezing a liquid, emulsion or suspension containing calcitriol and optional additives. The frozen units then undergo a lyophilisation process in freeze dryers. During lyophilisation a multitude of air pockets are created which help absorb the saliva and aid disintegration of the layer, which means the layer can be absorbed quickly in the oral cavity.

According to the invention, the first formulation comprising naltrexone is in the form of a tablet and the second formulation comprising calcitriol is a lyophilised liquid solution, suspension or emulsion which is attached to the tablet, thus forming a single unit oral dose. The first formulation and second formulation are two separate, distinct parts of the single unit oral dose pharmaceutical composition. The second formulation is attached to the tablet by any known means. For example, the second formulation may coat the tablet, or it may be formed as a layer, film or wafer in connection with or on the tablet.

The skilled person may use any known method for producing the single unit oral dose comprising a first formulation and a second formulation.

According to the invention, the single unit oral dose pharmaceutical composition comprises a first formulation comprising naltrexone in the form of a tablet and a second formulation comprising calcitriol as a lyophilised liquid solution, suspension or emulsion which is attached to the tablet, thus forming a single unit oral dose. The first formulation comprising naltrexone in the form of a tablet is formulated for delayed release. By delayed release, it is meant that the naltrexone is released after it has been swallowed by the patient. This ensures that naltrexone is predominantly absorbed in the gastrointestinal tract from the oesophagus onwards i.e. in the stomach. Furthermore, the second formulation comprising calcitriol being a lyophilised liquid solution, suspension or emulsion which is attached to the tablet ensures that calcitriol is absorbed in the oral cavity.

According to the invention, the first formulation comprising naltrexone is formulated for release and/or absorption after the second formulation comprising calcitriol is released and/or absorbed. By this it is meant that the calcitriol is formulated such that it is released and/or absorbed by the patient before the naltrexone is released and/or absorbed by the patient.

According to an embodiment which is not according to the invention, a method of forming a single unit oral dose pharmaceutical composition comprises the following steps:
- Providing a first formulation comprising 0.01 to 10 mg naltrexone;
- Providing a second formulation in contact with the first formulation, wherein the second formulation is a solution, suspension or emulsion comprising 80 to 200 ug calcitriol;
- Optionally freezing the combination of the first formulation and second formulation;
- Lyophilising the combination of the first formulation and the second formulation.

According to an embodiment which is not claimed, a method of forming a single unit oral dose pharmaceutical composition according to the present invention comprises the steps of:
(i) Providing a first formulation comprising 0.01 to 10 mg naltrexone;
(ii) Compressing the first formulation into a tablet;
(iii) Providing a second formulation comprising 80 to 200 ug calcitriol, wherein the second formulation is a solution, suspension or emulsion;
(iv) Dosing the second formulation into a pre-formed mould and optionally freezing the second formulation:
(v) Adding the first formulation to the preformed mould, such that the first formulation is in contact with the second formulation;
(vi) Lyophilising the combination of the first formulation and the second formulation.

In a preferred embodiment, the first step is the formulation of a mixture comprising 0.01 to 10 mg naltrexone. This mixture may be made by any known method, such as dry granulation or wet granulation. This mixture may optionally comprise a filler, a glidant, a disintegrant and/or a lubricant. The mixture is compressed into a tablet.

In a next step, the second formulation comprising 80 to 200 ug calcitriol is formulated as a solution, suspension or emulsion. The second formulation optionally comprises a matrix forming agent and/or a sugar or sugar alcohol, preferably mannitol and gelatin.

Preferably, the second formulation is then dosed into a preformed mould. In a next step, the first formulation is attached to the second formulation and the combination of formulations is frozen. This may be achieved by placing the pre-formed tablet in the mould, in contact with the second formulation. The freezing step is preferably carried out rapidly, for example for a period of 1 to 10 minutes, using very low temperatures, for example less than - 70 °C. The combination of formulations can be frozen by any suitable method known in the art, but preferably it is cryogenically frozen.

The final step is the lyophilisation of the frozen combination of formulations.

Lyophilisation is also called freeze-drying. Preferably, the lyophilisation process is carried out in a freeze-drying chamber typically operating under vacuum of 0.1 to 1.0 mbar for a period of time from 180 to 500 minutes.

In a preferred embodiment, the single unit oral dose pharmaceutical composition has a weight ratio of calcitriol to naltrexone in the range of 1:1 to 10:1. This ratio range has been found to provide an excellent level of activity in the treatment of cancers.

The single unit oral dose composition may comprise a third active ingredient. Preferably the third active ingredient is present in a third formulation. The third formulation can be made according to the methods described above for the first and second formulations.

In a preferred embodiment, the third active ingredient is a cannabinoid. Preferably, the cannabinoid is selected from the list consisting of: cannabidiol, cannabidiolic acid, cannabinol, tetrahydrocannabivarin, arachidonoylethanolamine, cannabidivarin, 2-arachidonoylglycerol, cannabigerol, cannabivarin, cannabichromene, 2-arachidonoyl glyceryl ether, N-arachidonoyl dopamine, virodhamine, dronabinol, nabilone, rimonabant or combinations thereof.

In a preferred embodiment, the third active is a flavonoid. Preferably the flavonoid is a flavonoid found in cannabis, such as cannaflavin-A, cannaflavin-B, cannaflavin- C, quercetin, isovitexin, apigenin, beta-sitosterol, luteolin, orientin, catechin, vitexin, silymarin, Kaempferol or combinations thereof.

In a preferred embodiment, the third active is a terpene. Preferably the terpene is a terpene found in cannabis, such as limonene, linalool, myrcene, pinene, phytol, terpinolene, trans-nerolidol, valencene, humulene, geraniol, eucalyptol, delta 3 carene, caryophyllene, camphene, borneol, bisabolol or combinations thereof.

In a second aspect of the invention, there is provided a single unit oral dose pharmaceutical composition for use as a medicament comprising a first active ingredient in a first formulation and a second active ingredient in a second formulation;
wherein the first active ingredient is for absorption in the gastrointestinal tract from the oesophagus onwards;
wherein the second active ingredient is formulated for absorption in the oral cavity;
wherein the first active ingredient is naltrexone in an amount of 0.01 to 10 mg and the second active ingredient is calcitriol in an amount of 80 to 200 ug;
wherein the first formulation comprising naltrexone is in the form of a tablet and wherein the second formulation comprising calcitriol is a lyophilised liquid solution, suspension or emulsion which is attached to the tablet;
wherein the first formulation comprising naltrexone in the form of a tablet is formulated for delayed release; and
wherein the first formulation comprising naltrexone is formulated for release and/or absorption after the second formulation comprising calcitriol is released and/or absorbed.

The single unit oral dose pharmaceutical composition for use as a medicament has the features described above.

The single unit oral dose pharmaceutical composition for use as a medicament is particularly suitable for subjects having a reduced ability to metabolise vitamin D. This is because such subjects are unable to metabolise vitamin D in the normal way to form calcitriol. Administration of calcitriol in a single unit oral dose according to the invention overcomes this problem.

In a third aspect of the invention, there is provided a single unit oral dose pharmaceutical composition for use in the treatment of cancer within a subject comprising a first active ingredient in a first formulation and a second active ingredient in a second formulation;
wherein the first active ingredient is for absorption in the gastrointestinal tract from the oesophagus onwards;
wherein the second active ingredient is formulated for absorption in the oral cavity;
wherein the first active ingredient is naltrexone in an amount of 0.01 to 10 mg and the second active ingredient is calcitriol in an amount of 80 to 200 ug;
wherein the first formulation comprising naltrexone is in the form of a tablet and wherein the second formulation comprising calcitriol is a lyophilised liquid solution, suspension or emulsion which is attached to the tablet;
wherein the first formulation comprising naltrexone in the form of a tablet is formulated for delayed release; and
wherein the first formulation comprising naltrexone is formulated for release and/or absorption after the second formulation comprising calcitriol is released and/or absorbed.

The single unit oral dose pharmaceutical composition for use in the treatment of cancer within a subject has the features described above.

The single unit oral dose pharmaceutical compositions may be used as a treatment for cancer, but primarily it is used to boost the effect of anti-cancer agents.

As used herein, the term "subject" refers to any animal (for example, a mammal), including, but not limited to, humans, non-human primates, canines, felines, rodents, and the like, which is to be the recipient of a treatment in which a pharmaceutical composition comprising a single unit dose is to be used according to the present invention. Typically, the terms "subject" and "patient" are used interchangeably herein in reference to a human subject.

As used herein, the term "treatment" refers to the therapeutic measures that cure, slow down, and/or halt progression of a diagnosed pathologic condition or disorder.

In some instances, a subject is successfully "treated" for a tumour/cancer according to the present invention if the subject shows one or more of the following: a reduction in the number of, or complete absence of, cancer cells; a reduction in the tumour size; inhibition of, or an absence of, cancer cell infiltration into peripheral organs including, for example, the spread of cancer into soft tissue and bone; inhibition of, or an absence of, tumour metastasis; inhibition of, or an absence of, tumour growth; reduced morbidity and mortality; reduction in tumourigenicity, tumourigenic frequency, or tumourigenic capacity of a tumour; reduction in the number or frequency of cancer stem cells in a tumour; differentiation of tumourigenic cells to a non-tumourigenic state; or some combination of effects.

As used herein, the term "cancer" refers to any mass of tissue that results from excessive cell growth, proliferation and/or survival, either benign (noncancerous) or malignant (cancerous), including pre-cancerous lesions. The terms "cancer", "tumour" and "neoplasm" may be used interchangeably. Tumours and cancers include benign, malignant, metastatic and non-metastatic types, and include any stage (I, II, III, IV or V) or grade (G1, G2, G3, etc.) of tumour, or cancer, or metastasis that is progressing, worsening, stabilized or in remission.

The single unit oral dose pharmaceutical composition according to the present invention may be used to treat any cancer, for example carcinoma, lymphoma, blastoma, sarcoma, and leukaemia; more particular examples of such tumours/cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, melanoma, vulval cancer, thyroid cancer, hepatic carcinoma and various types of head and neck cancers.

In a preferred embodiment, the single unit oral dose pharmaceutical composition for use according to the present invention is used to treat a subject having liver cancer and/or kidney cancer. The invention is particularly beneficial for subjects with kidney and/or liver cancer because such subjects are likely to have impaired renal and liver function, and so are unable to metabolise vitamin D in the normal way to form CCT.

In a preferred embodiment, the single unit oral dose pharmaceutical composition for use in the treatment of cancer is used for subjects having a reduced ability to metabolise vitamin D. This is because such subjects are unable to metabolise vitamin D in the normal way to form calcitriol. Certain cancer cells have elevated CYP enzymes which means vitamin D metabolism is compromised - specifically, CCT cannot be converted from its precursor CCD as it is unable to be converted from vitamin D. Administration of calcitriol in a single unit oral dose according to the invention overcomes this problem.

In a preferred embodiment, the single unit oral dose pharmaceutical composition is used for treating a subject who is undergoing or is selected to undergo treatment with an anti-cancer agent. The present inventors have discovered that the single unit dose of naltrexone and calcitriol according to the invention is a suitable booster or primer for anti-cancer agents, and thus allows the dose of the anti-cancer agents to be reduced.

As used herein, "anti-cancer agent" has its conventional meaning used in the art. For the purposes of the present invention the term chemotherapeutic agent is encompassed within the phrase "anti-cancer agent".

In a preferred embodiment, the anti-cancer agent may be selected from the group consisting of PI3-kinase inhibitors, AKT inhibitors, taxanes, antimetabolites, alkylating agents, cell cycle inhibitors, topoisomerase inhibitors and cytotoxic antibodies. The anti-cancer agent can be administered in any conventional way, the method of administration being largely dependent on the small molecule signalling inhibitor to be used. Accordingly, administration by *inter alia,* the parenteral, oral, sublingual, nasal and/or pulmonary routes are envisaged.

Where the anti-cancer agent is a PI3-kinase inhibitor, suitable examples include, but are not limited to, wortmannin, LY294002, demethoxyviridin, IC87114, NVP-BEZ235, BAY 80-6946, BKM120, GDC-0941, GDC-9080; including combinations thereof; and pharmaceutically acceptable salts, solvates, hydrates, stereoisomers, clathrates and prodrugs of any of the above.

Where the anti-cancer agent is an AKT inhibitor, suitable examples include, but are not limited to, MK-2206, GSK690693, perifosine, PHT-427, AT7867, honokiol, PF-04691502; including combinations thereof; and pharmaceutically acceptable salts, solvates, hydrates, stereoisomers, clathrates and prodrugs of any of the above.

Where the anti-cancer agent is a taxane, suitable examples include, but are not limited to, paclitaxel and docetaxel; including combinations thereof; and pharmaceutically acceptable salts, solvates, hydrates, stereoisomers, clathrates and prodrugs of any of the above.

Where the anti-cancer agent is an antimetabolite, suitable examples include, but are not limited to, methotrexate, 5-fluorouracil, capecitabin, cytosinarabinoside (Cytarabin), gemcitabine, 6-thioguanin, pentostatin, azathioprin, 6-mercaptopurin, fludarabin and cladribin; including combinations thereof; and pharmaceutically acceptable salts, solvates, hydrates, stereoisomers, clathrates and prodrugs of any of the above. Gemcitabine is an especially preferred antimetabolite. By way of example, gemcitabine may be administered at a dose (per administration) of 800-1200 mg/m², preferably 900-1100 mg/m², for example about 1000 mg/ m², or 1000 mg/m².

Where the anti-cancer agent is an alkylating agent, suitable examples include, but are not limited to, mechlorethamine, cyclophosphamide, ifosfamide, trofosfamide, melphalan (L-sarcolysin), chlorambucil, hexamethylmelamine, thiotepa, busulfan, carmustine (BCNU), streptozocin (streptozotocin), dacarbazine (DTIC; dimethyltriazenoimidazol ecarboxamide) temozolomide and oxaliplatin; including combinations thereof; and pharmaceutically acceptable salts, solvates, hydrates, stereoisomers, clathrates and prodrugs of any of the above. Cyclophosphamide and oxaliplatin are especially preferred alkylating agents. By way of example, oxaliplatin may be administered at a dose (per administration) of 65-105 mg/m², preferably 75-95 mg/m², for example about 85 mg/m², or 85 mg/m². By way of example, cyclophosphamide may be administered at a dose (per administration) of up to 1800 mg/m², for example 400-1800 mg/m².

Where the anti-cancer agent is a cell cycle inhibitor, suitable examples include, but are not limited to, Epothilone, Vincristine, Vinblastine, UCN-01, 17AAG, XL844, CHIR-124, PF-00477736, CEP-3891, Flavopiridol, berberine, P276-00, terameprocol, isoflavone daidzein, BI2536, BI6727, GSK461364, Cyclapolin, ON-01910, NMS-P937, TAK-960, Ispinesib, Monastrol, AZD4877, LY2523355, ARRY-520, MK-0731, SB743921, GSK923295, Lonafarnib, proTAME, Bortezomib, MLN9708, ONX0912, CEP-18770; including combinations thereof; and pharmaceutically acceptable salts, solvates, hydrates, stereoisomers, clathrates and prodrugs of any of the above; particularly suitable examples of cell cycle inhibitors include, but are not limited to, Hespaeradin, ZM447439, VX-680, MLN-8054, PHA-739358, AT-9283, AZD1152, MLN8237, ENMD2076, SU6668; including combinations thereof; and other inhibitors of Aurora kinases; and pharmaceutically acceptable salts, solvates, hydrates, stereoisomers, clathrates and prodrugs of any of the above.

In certain embodiments, the anti-cancer agent is an antimetabolite, preferably gemcitabine.

In certain embodiments, the anti-cancer agent is an alkylating agent, preferably oxaliplatin.

In a preferred embodiment, the anti-cancer agent may be a checkpoint inhibitor. A "checkpoint inhibitor" is an agent which acts on surface proteins which are members of either the TNF receptor or B7 superfamilies, including agents which bind to negative co-stimulatory molecules selected from CTLA-4, PD-1, TIM-3, BTLA, VISTA, LAG-3, and/or their respective ligands, including PD-L1. (Mellman et al., *supra).*

In a preferred embodiment, the anti-cancer agent is a cannabinoid. Preferably, the cannabinoid is selected from the list consisting of: cannabidiol, cannabidiolic acid, cannabinol, tetrahydrocannabivarin, arachidonoylethanolamine, cannabidivarin, 2-arachidonoylglycerol, cannabigerol, cannabivarin, cannabichromene, 2-arachidonoyl glyceryl ether, N-arachidonoyl dopamine, virodhamine, dronabinol, nabilone, rimonabant or combinations thereof.

In a preferred embodiment, the anti-cancer agent is a flavonoid. Preferably the flavonoid is a flavonoid found in cannabis, such as cannaflavin-A, cannaflavin-B, cannaflavin- C, quercetin, isovitexin, apigenin, beta-sitosterol, luteolin, orientin, catechin, vitexin, silymarin, Kaempferol or combinations thereof.

In a preferred embodiment, the anti-cancer agent is a terpene. Preferably the terpene is a terpene found in cannabis, such as limonene, linalool, myrcene, pinene, phytol, terpinolene, trans-nerolidol, valencene, humulene, geraniol, eucalyptol, delta 3 carene, caryophyllene, camphene, borneol, bisabolol or combinations thereof.

In a preferred embodiment, the single unit oral dose pharmaceutical composition is to be administered to the subject in a first treatment phase, and after the first treatment phase, the subject is to be administered a therapeutically effective amount of an anti-cancer agent in a second treatment.

In a preferred embodiment, the dosage regime of the single unit dose pharmaceutical composition for use according to the present invention is daily administration of a single unit dose.

Disclosed but not claimed is also the use of a single unit oral dose pharmaceutical composition for the manufacture of a medicament comprising a first active ingredient in a first formulation and a second active ingredient in a second formulation;
wherein the first active ingredient is for absorption in the gastrointestinal tract from the oesophagus onwards;
wherein the second active ingredient is formulated for absorption in the oral cavity;
wherein the first active ingredient is naltrexone in an amount of 0.01 to 10 mg and the second active ingredient is calcitriol in an amount of 80 to 200 ug;
wherein the first formulation comprising naltrexone is in the form of a tablet and wherein the second formulation comprising calcitriol is a lyophilised liquid solution, suspension or emulsion which is attached to the tablet;
wherein the first formulation comprising naltrexone in the form of a tablet is formulated for delayed release; and
wherein the first formulation comprising naltrexone is formulated for release and/or absorption after the second formulation comprising calcitriol is released and/or absorbed.

This single unit oral dose pharmaceutical composition has the features described above.

Disclosed but not claimed is also the use of a single unit oral dose pharmaceutical composition for the manufacture of a medicament for use in the treatment of cancer comprising a first active ingredient in a first formulation and a second active ingredient in a second formulation;
wherein the first active ingredient is for absorption in the gastrointestinal tract from the oesophagus onwards;
wherein the second active ingredient is formulated for absorption in the oral cavity;
wherein the first active ingredient is naltrexone in an amount of 0.01 to 10 mg and the second active ingredient is calcitriol in an amount of 80 to 200 ug;
wherein the first formulation comprising naltrexone is in the form of a tablet and wherein the second formulation comprising calcitriol is a lyophilised liquid solution, suspension or emulsion which is attached to the tablet;
wherein the first formulation comprising naltrexone in the form of a tablet is formulated for delayed release; and
wherein the first formulation comprising naltrexone is formulated for release and/or absorption after the second formulation comprising calcitriol is released and/or absorbed.

This single unit oral dose pharmaceutical composition has the features described above.

### Examples

### Example 1: Synthesis of a single unit dose according to the invention

A single unit dose pharmaceutical composition according to a preferred embodiment of the invention was made as follows:
A tablet containing naltrexone (LDN) was formulated with the following components:

| **Component** | **Quantity per low strength (1mg) Tablet (mg)** | **Quantity per high strength (10mg) Tablet (mg)** | **Function** | **Reference to Standard** |
|---|---|---|---|---|
| Naltrexone Hydrochloride¹ | 1.00 | 10.00 | Active | Ph. Eur, USP |
| Microcrystalline Cellulose | 91.30 | 82.30 | Filler | Ph. Eur., JP, USP/NF |
| Pregelatinised Starch | 5.00 | 5.00 | Filler | Ph. Eur., USP/NF |
| Crospovidone | 2.00 | 2.00 | Disintegrant | Ph. Eur., JP, USP/NF |
| Silica, Colloidal Anhydrous | 0.20 | 0.20 | Glidant | Ph. Eur., USP/NF |
| Magnesium Stearate | 0.50 | 0.50 | Lubricant | Ph. Eur., JP, USP/NF |
| **Total per tablet** | **100.00** | **100.00** | | |

The formulation was initially a dry mix which was pressed into a 100 mg tablet.

The tablet has a crystalline coating, onto which the CCT formulation is attached. The CCT formulation was formulated from a liquid solution or suspension containing 140 ug CCT, gelatin and mannitol.

The liquid/suspension was added to pre-formed blisters and then the above described LDN tablet was attached to form the single unit dose. It was then passed through a cryogenic freezing process. The frozen units then undergo lyophilisation process in freeze dryers. The blisters containing the dried units are then sealed via a heat-seal process to protect the product from varying environmental conditions and ensure long-term stability.

### Reference Example 2: Administration of naltrexone (LDN) and calcitriol (CCT) in combination with chemotherapeutic agents

Cancer cell lines A549 (Lung) or HCT116 (Colon) were reset at a density of 1.5 x 10⁷4/ml and allowed to adhere to 6-well plates for 4h. Cells were then subject to a regimen that consisted of two treatment phases each lasting 48h. In phase 1, cells were either cultured with 10 nM naltrexone (LDN) or with 10 nM naltrexone and 10 nM CCT (LDN + CCT or LDNC). At the end of this first phase, the exhausted media were replaced with fresh media. The second phase of treatment was then applied which was composed of either 1 uM gemcitabine (GEM) or 500 nM oxaliplatin (OXP). At the end of the treatments, cells were harvested with trypsin, and cell number and percentage viability assessed by cell counting using trypan blue dye exclusion to aid discrimination of non-viable cells.

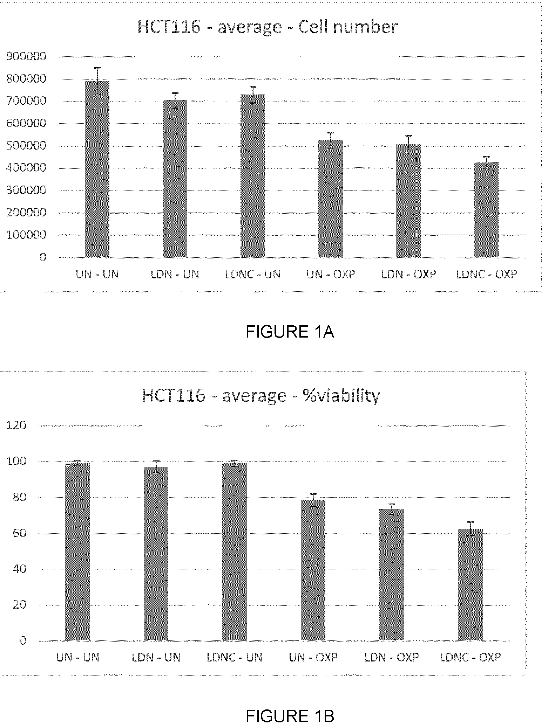

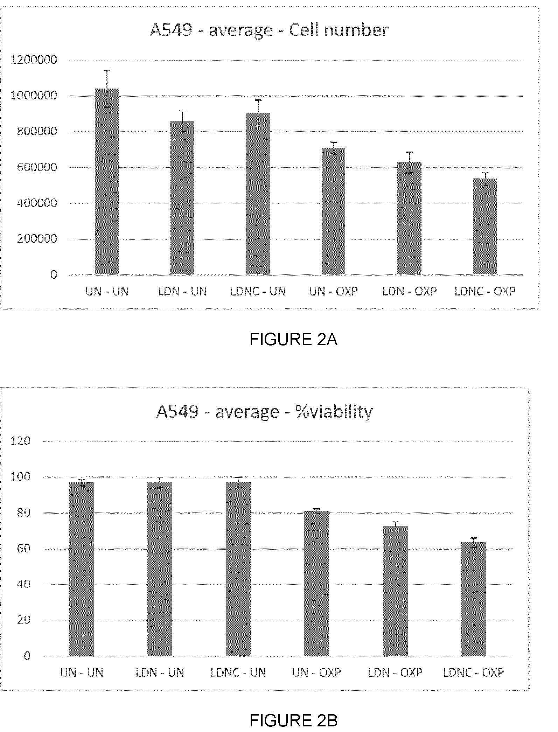

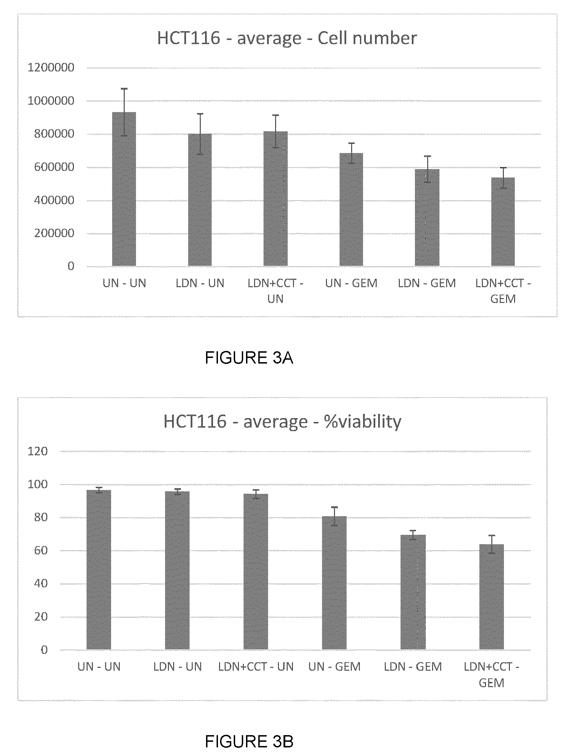

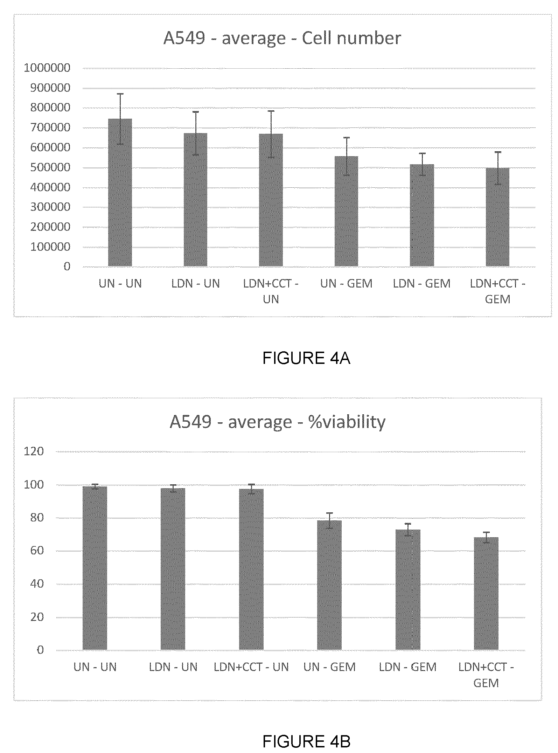

The experiments show that the combination of naltrexone and calcitriol is more effective in boosting the effect of chemotherapeutic agents gemcitabine and oxaliplatin, than naltrexone alone (see Figures 1A, 1B, 2A, 2B, 3A, 3B, 4A, 4B).

## Claims

1. A single unit oral dose pharmaceutical composition comprising a first active ingredient in a first formulation and a second active ingredient in a second formulation;
wherein the first active ingredient is for absorption in the gastrointestinal tract from the oesophagus onwards;
wherein the second active ingredient is formulated for absorption in the oral cavity;
wherein the first active ingredient is naltrexone in an amount of 0.01 to 10 mg and the second active ingredient is calcitriol in an amount of 80 to 200 ug;
wherein the first formulation comprising naltrexone is in the form of a tablet and wherein the second formulation comprising calcitriol is a lyophilised liquid solution, suspension or emulsion which is attached to the tablet;
wherein the first formulation comprising naltrexone in the form of a tablet is formulated for delayed release; and
wherein the first formulation comprising naltrexone is formulated for release and/or absorption after the second formulation comprising calcitriol is released and/or absorbed.

2. The single unit oral dose pharmaceutical composition according to claim 1, comprising 0.01 to 6 mg of naltrexone, more preferably between 3 to 4.5 mg.

3. The single unit oral dose pharmaceutical composition according to claim 2, wherein the second formulation comprising calcitriol is formulated for sublingual absorption.

4. The single unit dose pharmaceutical composition according to any preceding claim, wherein the weight ratio of calcitriol to naltrexone is in the range of 1:1 to 10:1.

5. The single unit dose pharmaceutical composition according to any preceding claim, further comprising a cannabinoid, flavonoid or terpene, preferably selected from the list consisting of: cannabidiol, cannabidiolic acid, cannabinol, tetrahydrocannabivarin, arachidonoylethanolamine, cannabidivarin, 2-arachidonoylglycerol, cannabigerol, cannabivarin, cannabichromene, 2-arachidonoyl glyceryl ether, N-arachidonoyl dopamine, virodhamine, dronabinol, nabilone, rimonabant, cannaflavin-A, cannaflavin-B, cannaflavin-C, quercetin, isovitexin, apigenin, beta-sitosterol, luteolin, orientin, catechin, vitexin, silymarin, Kaempferol, limonene, linalool, myrcene, pinene, phytol, terpinolene, trans-nerolidol, valencene, humulene, geraniol, eucalyptol, delta 3 carene, caryophyllene, camphene, borneol, bisabolol or combinations thereof.

6. A single unit oral dose pharmaceutical composition according to any of claims 1 to 5 for use as a medicament.

7. A single unit oral dose pharmaceutical composition according to any of claims 1 to 5 for use in the treatment of cancer within a subject.

8. The single unit oral dose pharmaceutical composition for use according to claim 7, wherein the subject is undergoing or is selected to undergo treatment with an anti-cancer agent.

9. The single unit oral dose pharmaceutical composition for use according to claim 7 or claim 8, wherein the subject has liver cancer and/or kidney cancer and/or wherein the subject has a reduced ability to metabolise vitamin D.

10. The single unit oral dose pharmaceutical composition for use according to claims 7 to 9, wherein the single unit dose pharmaceutical composition is to be administered to the subject in a first treatment phase, and wherein after the first treatment phase, the subject is to be administered a therapeutically effective amount of an anti-cancer agent in a second treatment.

11. The single unit dose pharmaceutical composition for use according to claims 7 to 10, wherein the dosage regime is daily administration of a single unit dose.

## Patentansprüche

1. Pharmazeutische orale Einzeldosis-Zusammensetzung, die einen ersten Wirkstoff in einer ersten Formulierung und einen zweiten Wirkstoff in einer zweiten Formulierung umfasst;
wobei der erste Wirkstoff zur Absorption im Gastrointestinaltrakt ab dem Oesophagus dient;
wobei der zweite Wirkstoff zur Absorption in der Mundhöhle formuliert ist;
wobei der erste Wirkstoff Naltrexon in einer Menge von 0,01 bis 10 mg und der zweite Wirkstoff Calcitriol in einer Menge von 80 bis 200 ug ist;
wobei die erste Naltrexon umfassende Formulierung in der Form einer Tablette ist, und wobei die zweite Calcitriol umfassende Formulierung eine lyophilisierte flüssige Lösung, Suspension oder Emulsion ist, die an der Tablette angebracht ist;
wobei die erste Naltrexon umfassende Formulierung in der Form einer Tablette für verzögerte Freigabe formuliert ist; und
wobei die erste Naltrexon umfassende Formulierung zur Freigabe und/oder Absorption nachdem die der zweite Calcitriol umfassende Formulierung freigegeben und/oder absorbiert ist formuliert ist.

2. Pharmazeutische orale Einzeldosis-Zusammensetzung nach Anspruch 1, umfassend 0,01 bis 6 mg Naltrexon, besonders bevorzugt zwischen 3 bis 4,5 mg.

3. Pharmazeutische orale Einzeldosis-Zusammensetzung nach Anspruch 2, wobei die zweite Calcitriol umfassende Formulierung zur sublingualen Absorption formuliert ist.

4. Pharmazeutische Einzeldosis-Zusammensetzung nach einem vorstehenden Anspruch, wobei das Gewichtsverhältnis von Calcitriol zu Naltrexon im Bereich von 1:1 bis 10:1 liegt.

5. Pharmazeutische Einzeldosis-Zusammensetzung nach einem vorstehenden Anspruch, weiter umfassend ein Cannabinoid, Flavonoid oder Terpen, vorzugsweise ausgewählt aus der Liste bestehend aus: Cannabidiol, Cannabidiolsäure, Cannabinol, Tetrahydrocannabivarin, Arachidonoylethanolamin, Cannabidivarin, 2- Arachidonylglycerol, Cannabigerol, Cannabivarin, Cannabichromen, 2-Arachidonylglycerolether, N-Arachidonoyldopamin, Virodhamin, Dronabinol, Nabilon, Rimonabant, Cannaflavin-A, Cannaflavin-B, Cannaflavin-C, Quercetin, Isovitexin, Apigenin, Beta-Sitosterin, Luteolin, Orientin, Catechin, Vitexin, Silymarin, Kaempferol, Limonen, Linalool, Myrcen, Pinen, Phytol, Terpinolen, Trans-Nerolidol, Valencen, Humulen, Geraniol, Eucalyptol, Delta-3-Caren, Caryophyllen, Camphen, Borneol, Bisabolol oder Kombination daraus.

6. Pharmazeutische orale Einzeldosis-Zusammensetzung nach einem der Ansprüche 1 bis 5 zur Verwendung als ein Medikament.

7. Pharmazeutische orale Einzeldosis-Zusammensetzung nach einem der Ansprüche 1 bis 5 zur Verwendung in der Behandlung von Krebs innerhalb eines Subjekts.

8. Pharmazeutische orale Einzeldosis-Zusammensetzung zur Verwendung nach Anspruch 7, wobei das Subjekt sich einer Behandlung mit einem Mittel gegen Krebs unterzieht oder ausgewählt wurde, um sich einer solchen zu unterziehen.

9. Pharmazeutische orale Einzeldosis-Zusammensetzung zur Verwendung nach Anspruch 7 oder Anspruch 8, wobei das Subjekt Leberkrebs und/oder Nierenkrebs aufweist und/oder wobei das Subjekt verringerte Fähigkeit für den Vitamin-D-Stoffwechsel aufweist.

10. Pharmazeutische orale Einzeldosis-Zusammensetzung zur Verwendung nach den Ansprüchen 7 bis 9, wobei die pharmazeutische orale Einzeldosis-Zusammensetzung dem Subjekt in einer ersten Behandlungsphase zu verabreichen ist, und wobei nach der ersten Behandlungsphase dem Subjekt eine therapeutisch wirksame Menge eines Mittels gegen Krebs in einer zweiten Behandlung zu verabreichen ist.

11. Pharmazeutische orale Einzeldosis-Zusammensetzung zur Verwendung nach den Ansprüchen 7 bis 10, wobei das Dosierungsschema tägliche Verabreichung einer Einzeldosis ist.

## Revendications

1. Composition pharmaceutique monodose par voie orale comprenant un premier principe actif dans une première formulation et un second principe actif dans une seconde formulation ;
dans laquelle le premier principe actif est destiné à être absorbé dans le tractus gastrointestinal à partir de l'oesophage et au-delà ;
dans laquelle le second principe actif est formulé pour une absorption dans la cavité orale ; dans laquelle le premier principe actif est de la naltrexone dans une quantité de 0,01 à 10 mg et le second principe actif est du calcitriol dans une quantité de 80 à 200 µg ;
dans laquelle la première formulation comprenant la naltrexone est sous la forme d'un comprimé et dans laquelle la seconde formulation comprenant le calcitriol est une solution liquide, suspension ou émulsion lyophilisée qui est liée au comprimé ;
dans laquelle la première formulation comprenant la naltrexone sous la forme d'un comprimé est formulée pour une libération retardée ; et
dans laquelle la première formulation comprenant la naltrexone est formulée pour une libération et/ou une absorption une fois la seconde formulation comprenant le calcitriol libérée et/ou absorbée.

2. Composition pharmaceutique monodose par voie orale selon la revendication 1, comprenant 0,01 à 6 mg de naltrexone, de préférence entre 3 à 4,5 mg.

3. Composition pharmaceutique monodose par voie orale selon la revendication 2, dans laquelle la seconde formulation comprenant le calcitriol est formulée pour une absorption sublinguale.

4. Composition pharmaceutique monodose selon une quelconque revendication précédente, dans laquelle le rapport de poids du calcitriol par rapport à la naltrexone est dans la plage de 1:1 à 10:1.

5. Composition pharmaceutique monodose selon une quelconque revendication précédente, comprenant en outre un cannabinoïde, un flavonoïde ou un terpène, de préférence choisi dans la liste consistant en: cannabidiol, acide cannabidiolique, cannabinol, tétrahydrocannabivarine, arachidonoyl-éthanolamine, cannabidivarine, 2-arachidonoylglycérol, cannabigérol, cannabivarine, cannabichromène, 2-arachidonoyl glycéryl éther, N-arachidonoyl dopamine, virodhamine, dronabinol, nabilone, rimonabant, cannflavine A, cannflavine B, cannflavine C, quercétine, isovitexine, apigénine, bêta-sitostérol, lutéoline, orientine, catéchine, vitexine, silymarine, kaempférol, limonène, linalool, myrcène, pinène, phytol, terpinolène, trans-nérolidol, valencène, humulène, géraniol, eucalyptol, delta-3-carène, caryophyllène, camphène, bornéol, bisabolol ou des combinaisons de ceux-ci.

6. Composition pharmaceutique monodose par voie orale selon l'une quelconque des revendications 1 à 5 destinée à être utilisée comme médicament.

7. Composition pharmaceutique monodose par voie orale selon l'une quelconque des revendications 1 à 5 destinée à être utilisée dans le traitement du cancer chez un sujet.

8. Composition pharmaceutique monodose par voie orale destinée à être utilisée selon la revendication 7, dans laquelle le sujet suit ou a été choisi pour suivre un traitement avec un agent anti-cancer.

9. Composition pharmaceutique monodose par voie orale destinée à être utilisée selon la revendication 7 ou la revendication 8, dans laquelle le sujet présente un cancer du foie et/ou un cancer du rein et/ou dans laquelle le sujet présente une capacité réduite à métaboliser la vitamine D.

10. Composition pharmaceutique monodose par voie orale destinée à être utilisée selon les revendications 7 à 9, dans laquelle la composition pharmaceutique monodose doit être administrée au sujet dans une première phase de traitement, et dans laquelle, après la première phase de traitement, une quantité thérapeutiquement efficace d'un agent anti-cancer doit être administrée au sujet dans un second traitement.

11. Composition pharmaceutique monodose destinée à être utilisée selon les revendications 7 à 10, dans laquelle le régime posologique est une administration quotidienne d'une monodose.
